# EUROPEAN PATENT APPLICATION

(11) **EP 3 738 646 A1**
(43) Date of publication of application: **18.11.2020**
(21) Application number: 19912201.1
(22) Date of filing: 16.04.2019
(51) Int. Cl.: A61N 1/36, A61H 39/00

(54) **NECK MASSAGER**

(30) Priority: 12.03.2019 CN 201910187757; 12.03.2019 CN 201910187732
(71) Applicant: Wear Future Technologies Co., Ltd, Shenzhen, Guangdong 518000 (CN)
(72) Inventor: LIU, Jie, Shenzhen, Guangdong 518000 (CN); WANG, Zhiguo, Shenzhen, Guangdong 518000 (CN); XIAO, Hua, Shenzhen, Guangdong 518000 (CN)
(74) Representative: karo IP
(86) International application number: PCT/CN2019/082809
(87) International publication number: WO 2020/181611

(57) **Abstract**

A neck massaging device includes an elastic arm, two handles, an electrode assembly, an electric pulse generating device and a hollow flexible sleeve, the two handles connected to each end of the carrying body, the electric pulse generating device electrically connected with the electrode assembly, the electric pulse generating device arranged in one of the handles, the flexible sleeve tensioned over the carrying body, the electrode assembly disposed on the flexible sleeve.

## Description

The present disclosure claims priority to the Chinese Patent Application No. 201910187757.9, filed to the Patent Office of the People's Republic of China on 12 March, 2019 and entitled "NECK MASSAGING DEVICE, which is incorporated in the present disclosure by reference in its entity.

The present disclosure claims priority to the Chinese Patent Application No. 201910187732.9, filed to the Patent Office of the People's Republic of China on 12 March, 2019 and entitled "ELECTRODE PLATE MOUNTING STRUCTURE AND NECK MASSAGING DEVICE, which is incorporated in the present disclosure by reference in its entity.

### FIELD

The present disclosure relates to a massaging device and, more particularly, to a neck massaging device.

### BACKGROUND

In recent years, due to work and bad living habits, cervical spondylosis has youth-oriented tendency, and thus, a neck massaging device appears at the right moment.

The applicant previously proposes a neck-hanging massaging device including an elastic arm, a first handle, a second handle, an electrode assembly, and an electric pulse generating device electrically connected with the electrode assembly. The first handle and the second handle are fixed to two sides of the elastic arm. The electrode assembly is arranged on the elastic arm. The electric pulse generating device is arranged in the first handle. The elastic arm includes a plastic outer shell and a silicone rubber inner shell which are bonded together through glue.

In a manufacturing process, it is found that glue overflowing, due to uneven painting glue, often occur when bonding the plastic outer shell with the silicone rubber inner shell. Therefore, the overflowing glue has to be wiped off and extra time has to be spent. In addition, it may be ensured that all portions between the plastic outer shell and the silicone rubber inner shell are bonded firmly, thus resulting a low assembling efficiency of a product. Moreover, it is possible that degumming occurs between the plastic outer shell and the silicone rubber inner shell, which may be resulted by factors such as ageing of the glue after a period of time, which affects the consumption experience.

### SUMMARY

The present disclosure is mainly aimed to provide a neck massaging device with high assembly efficiency and good consumption experience.

According to an embodiment of the present disclosure, a neck massaging device may be provided, which may include a carrying body, two handles, an electrode assembly, a flexible sleeve which is hollow and an electric pulse generating device, the two handles connected to each end of the carrying body, the electric pulse generating device electrically connected with the electrode assembly, the electric pulse generating device arranged in one of the handles, the flexible sleeve tensioned over the carrying body, the electrode assembly disposed on the flexible sleeve.

Alternatively, the carrying body may be configured into an arc-shaped sheet body and have two long sides and two short sides. The carrying body may be provided with clamping strips extending along the two long sides of the carrying body, and the flexible sleeve may be correspondingly provided with clamping grooves in which the clamping strips are embedded.

Alternatively, there are two clamping strips. The two clamping strips may be symmetrically distributed with each being arranged on each long side respectively.

Alternatively, the two short sides may be provided with two mounting heads respectively. The pair of handles may be fixed to the two mounting heads respectively.

Alternatively, each of the handles may include an outer shell threadedly connected to the mounting head, and an inner shell buckled with the outer shell, and further detachably clamped on each mounting head.

Alternatively, a locking block may be protruded on the mounting head, and the flexible sleeve is correspondingly provided with a locking groove in which the locking blocks are locked

Alternatively, the outer shell may be provided with a grabbing buckle, and a buckle hole may be correspondingly formed in the flexible sleeve; wherein the grabbing buckle is hooked in the buckle hole.

Alternatively, a stepped structure may be formed on each end of the flexible sleeve. The neck massaging device may further include a decorative ring hooked over the stepped structure and borne against a position between the flexible sleeve and one of the handles.

Alternatively, the flexible sleeve is integrally formed with a mounting boss, on which the electrode assembly is mounted. The electrode assembly may swing freely by 360-degree in a floating manner via the folding deformation of the mounting boss.

Alternatively, the carrying body is an anti-deformation member or an elastic arm.

According to some embodiments of the present disclosure, an electrode plate mounting structure may include a carrying body, a hollow flexible sleeve tensioned over the carrying body; and electrode plates arranged on the flexible sleeve.

Alternatively, mounting bosses may be disposed integrally on the flexible sleeve, on which the electrode plates are mounted respectively. The electrode plates may swing freely by 360-degree in a floating manner via the folding deformation of the mounting boss.

Alternatively, the carrying body is an anti-deformation member or an elastic arm.

Alternatively, the carrying body may be configured into an arc-shaped sheet body and have two long sides and two short sides. The carrying body may be provided with clamping strips extending along the two long sides of the carrying body, and the flexible sleeve may be correspondingly provided with clamping grooves in which the clamping strips are embedded.

Alternatively, the number of the clamping strips may be two. The two clamping strips may be symmetrically distributed with each being arranged on each long side respectively.

According to the neck massaging device of the present disclosure, by arranging the flexible sleeve to replace the silicon rubber inner shell, which may be directly sleeved on the elastic arm without an additional bonding way, a series of problems brought by glue overflowing mentioned in the background may be avoided, and thus, the assembling efficiency and consumption experience are greatly improved.

### BRIEF DESCRIPTION OF THE DRAWINGS

In order to explain the technical solutions in the embodiments of the present disclosure or the prior art more clearly, the drawings used by the description of the embodiments or the prior art will be briefly introduced below. Obviously, the drawings in the following description may be merely some embodiments of the present disclosure. For those of ordinary skilled in the art, other drawings may be obtained according to the structures shown in the drawings without creative effort, in which:
Fig. 1 is a perspective view of a neck massaging device according to an embodiment of the present disclosure;
Fig. 2 is a front view of the neck massaging device as shown in Fig. 1;
Fig. 3 is an exploded perspective view of one of handles, decorative rings, an elastic arm and the like as shown in Fig. 1;
Fig. 4 is an exploded perspective view from another angle of one of the handles, the decorative rings, the elastic arm and the like as shown in Fig. 1;
Fig. 5 is a perspective view of the elastic arm as shown in Fig. 1;
Fig. 6 is a perspective view of a flexible sleeve as shown in Fig. 1;
Fig. 7 is a sectional perspective view of the flexible sleeve as shown in Fig. 5;
Fig. 8 is a schematic mounting view of the elastic arm, the flexible sleeve and an electrode assembly as shown in Fig. 1.

### DETAILED DESCRIPTION

In the following, the technical solutions in the embodiments of the present disclosure will be clearly and completely described with reference to the drawings in the embodiments of the present disclosure. Obviously, the described embodiments may be only a part of the embodiments of the present disclosure, but not all of the embodiments. Based on the embodiments of the present disclosure, all other embodiments obtained by those of ordinary skilled in the art without creative effort shall fall within the protection scope of the present disclosure.

It should be noted that all directional indications (such as up, down, left, right, front, and rear ... ... ) in the embodiments of the present invention are only used to explain the relative position relationship between the components, the movement situation, and the like in a specific posture (as shown in the drawings), and if the specific posture is changed, the directional indication is changed accordingly.

In addition, descriptions such as "first" and "second" in the present disclosure may be for descriptive purposes only, and cannot be understood as indicating or implying the relative importance or implicitly indicating the number of indicated technical features. Therefore, the features defined as "first" and "second" may explicitly or implicitly include at least one of the features. In the description of the present disclosure, the meaning of "a plurality of' may be at least two, for example, two, and three and so on, unless it may be specifically defined otherwise.

According to the present disclosure, the terms "connection", "fixing" and the like should be understood in a broad sense unless otherwise specified and defined, for example, "fixing" may be fixed connection or detachable connection or an integral whole, may be mechanical connection or electrical connection, may be direct connection or indirect connection through an intermediate medium, and may be internal connection of two elements or interaction between two elements, unless it may be clearly defined otherwise. For those of ordinary skilled in the art, the specific meanings of the above terms in the present disclosure may be understood according to specific situations.

In addition, the technical solutions between the various embodiments of the present disclosure may be combined with each other, but must be based on what may be achieved by those of ordinary skilled in the art. When the combination of technical solutions conflicts or cannot be achieved, such a combination of technical solutions should be considered to be nonexistent and not within the scope of protection claimed by the present disclosure.

A neck massaging device according to an embodiment of the present disclosure will be described.

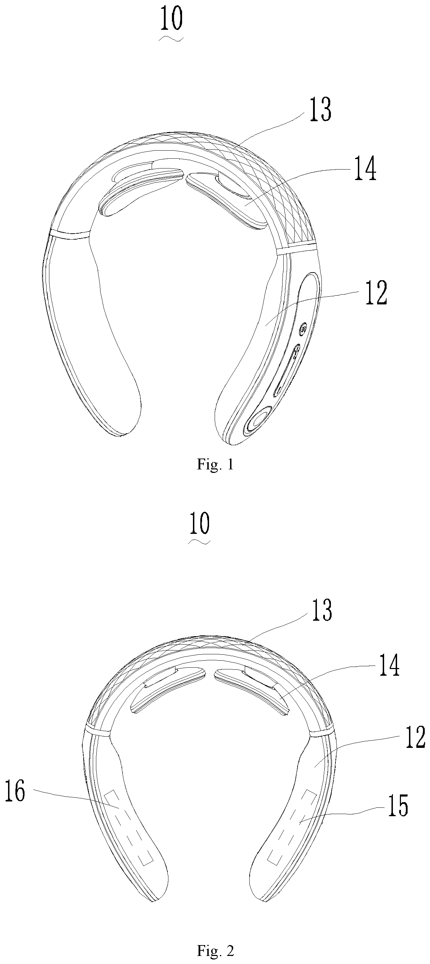

As shown in Fig.1 to Fig.5, the neck massaging device 10 may include a carrying body 11, a pair of handles 12, a hollow flexible sleeve 13, an electrode assembly 14, an electric pulse generating device 15 and a battery 16. A pair of handles 12 may be fixed to each end of the carrying body 11. The electric pulse generating device 15 may be arranged in one of the handles 12 and may be electrically connected with the electrode assembly 14. The battery 16 may be arranged in the other handle 12. The flexible sleeve 13 may be tensioned over the carrying body 11. The electrode assembly 14 is arranged on the flexible sleeve 13.

It should be noted that the flexibility of the flexible sleeve 13 is relative to the rigidity, for example, of a plastic shell. Therefore, it will be appreciated that the expression of 'flexibility' does not bring about the defect of unclear specification.

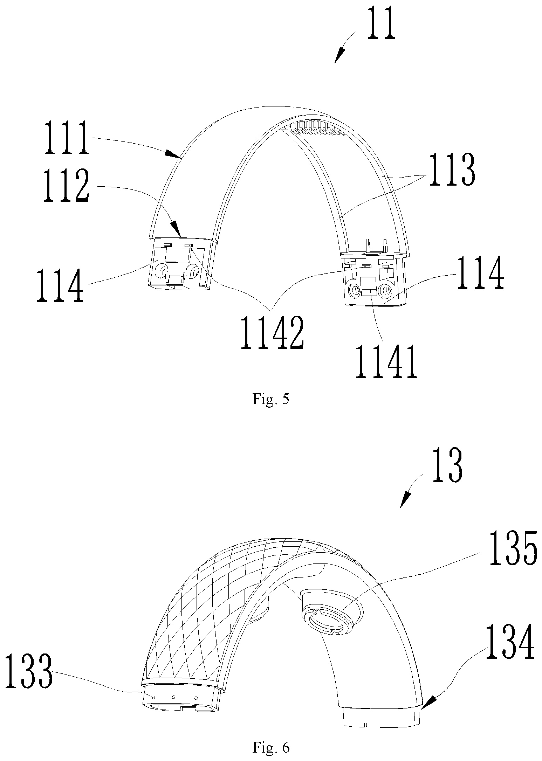

As shown in Figs.5-8, the carrying body 11 may include two long sides 111 and two short sides 112. The carrying body 11 may be provided with clamping strips 113 extending along the two long sides 111 of the carrying body 11. And the flexible sleeve 13 may be correspondingly provided with clamping grooves 131 in which the clamping strips 113 are embedded. In this way, the degree of freedom that the flexible sleeve 13 rotates around the long sides 113 is limited, namely the displacement of the flexible sleeve 13 caused by its rotating around the carrying body 11 may be avoided. Therefore, it may be ensured that the position of the electrode assembly 14 on the flexible sleeve 13 is stable. In an alternative implementation, the carrying body 11 may be an elastic arm. The neck massaging device 10 may be clamped on the neck of a wearer by the deformation of the carrying body 11.

In some further embodiment, the flexible sleeve 13 may be formed as arc-shaped, and the carrying body 11 may also be formed arc-shaped. The curvature of the flexible sleeve 13 may correspond to the curvature of the carrying body 11. Such a design is based on the principle: when the curvature of the flexible sleeve 13 is not too consistent with the curvature of the carrying body 11, the tensioning degrees of all portions of the flexible sleeve 13 over the carrying body 11 may be very different, which may affect the aesthetic appearance of the product and in severe cases, may also result in localized loose shifting.

In an alternative implementation, the number of the clamping strips 113 may be two. The two clamping strips 113 may be respectively arranged on the long side 111. The two clamping strips 113 may be symmetrically distributed. In this way, the flexible sleeve 13 may relatively smoothly sleeve the carrying body 11.

Referring to Fig.1, Fig.3 and Fig.5, two short sides 112 are provided with two mounting heads respectively. The two handles are fixed to the two mounting head 114 respectively. The handles 12 are directly arranged on the mounting heads 114 of the carrying body 11 without a third-party structural element, so that the cost may be reduced, and the installation efficiency improved.

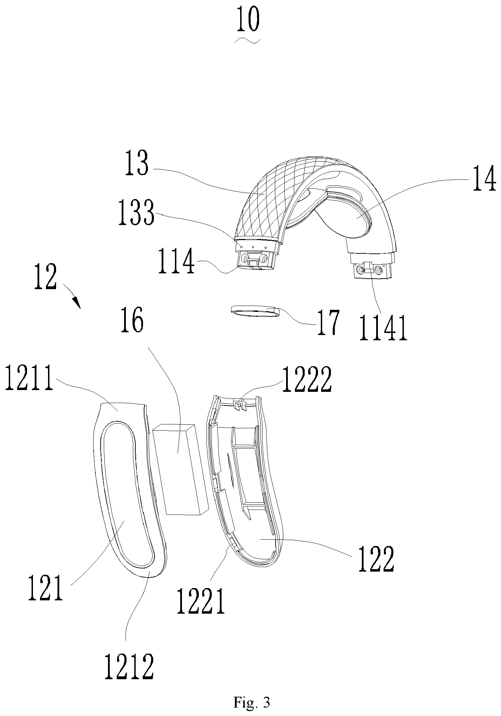

As shown in Figs.3-4, each of the handles 12 may include an outer shell 121 and an inner shell 122 buckled with the outer shell 121. The outer shell 121 may include a connecting end 1211 and a free end 1212 opposite to the connecting end 1211. The connecting end 1211 may be connected to each mounting head 114. The connecting end 1211 may be slightly bent. When each handle 12 is forced to be outwards pulled apart, the connecting end 1211 may be slightly deformed to force the free end 1212 to slightly swing with the mounting head 114 as a pivot. Alternatively, the outer shell 121 may be further provided with a plurality of clamping holes 1213 surrounding the battery 16, and the inner shell 122 may be correspondingly provided with clamping hooks 1221. The outer shell 121 and the inner shell 122 are connected by engaging the clamping hooks 1221 to the clamping holes 1213. In addition, the inner shell 122 may be provided with clamping block 1222. Correspondingly, clamping port 1141 detachably engaging to the clamping block 1222 may be disposed on the mounting heads 114. The position of the clamping block 1222 may be arranged correspond to the connecting end 1211 of the outer shell 121.

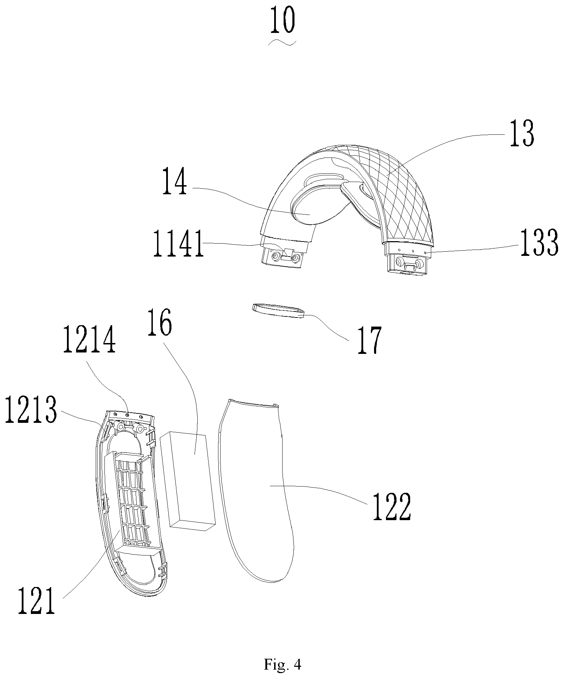

According to a further embodiment of the present disclosure, as shown in Figs. 4-7, a locking block 1142 may be protruded on the mounting head 114. Correspondingly, a locking groove 132 fitting with the locking block 1142 may be formed in the flexible sleeve 13. In this way, the degree of freedom of the flexible sleeve 13 in the extension directions of the long sides 111 may be limited, namely the displacement of the flexible sleeve 13 caused by its rotating around the carrying body 11 may be avoided. Therefore, it is ensured that the position of the electrode assembly 14 on the flexible sleeve 13 is stable.

As shown in Fig. 3 and Fig. 6, the outer shell 121 may be further provided with grabbing buckles 1214, and the flexible sleeve 13 is correspondingly provided with buckle holes 133. The grabbing buckles 1214 may be hooked into the buckle hole 133. In this way, the degree of freedom of the flexible sleeve 13 in the extension directions of the long sides 111 may be further limited. It should be noted that the grabbing buckle 1214 may be arranged on the outer shell 121 because the outer shell 121 has been already connected to the mounting heads 114 by screw, without simultaneously connected with the inner shell 122. Therefore, it is easier to hook the grabbing buckle 1214 in the buckle holes 133.

As shown in Fig.3, Fig.4 and Fig.7, the neck massaging device 10 may further include two decorative rings 17. Correspondingly, each end of the flexible sleeve 13 may be provided with a stepped structure 134. The decorative rings 17 may be covered on the stepped structures 134 and borne against a position between the flexible sleeve 13 and one of the handles 12. The decorative ring 17 may be of collar structure. It is ensured that the locking block 1142 is more stably locked in the locking groove 132. And the phenomenon that the locking block 1142 is separated from the locking groove 132 when the inner shell 122 is clamped on the outer shell 121 and the mounting head 114 may be avoided.

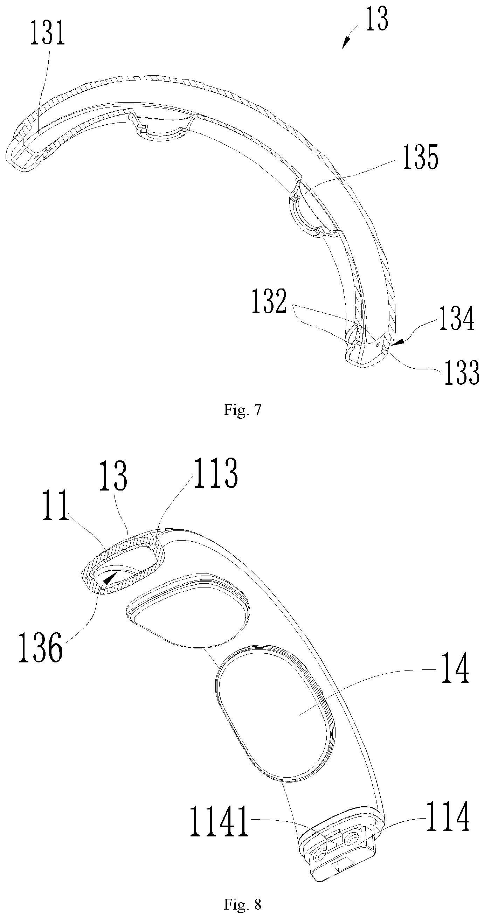

According to a further embodiment of the present disclosure, as shown in Figs.7-8, the flexible sleeve 1 may be integrally formed with a mounting boss 135. The electrode assembly 14 may be mounted on the mounting boss 135. The electrode assembly 14 may swings freely by 360-degree in a floating manner via the folding deformation of the mounting boss 135. In addition, a gap 136 may be formed when the hollow flexible sleeve 13 covers the carrying body 11, which may provide more convenience for the 360-degree floating swing of the electrode assembly 14.

Alternatively, the flexible sleeve 13 may be made of silicone rubber which is low in price and skin friendly.

When the neck massaging device 10 may be mounted, the flexible sleeve 13 may firstly be covered outside of the carrying body 11 with the two mounting heads 114 of the carrying body 11 exposed uniformly. The clamping strips 113 may be embedded in the clamping grooves 131 respectively, and the locking block 1142 may be locked in the locking groove 132 respectively. Secondly, the two decorative rings 17 may be respectively mounted to each mounting head 114. And then, the connecting ends 1211 of the outer shells 121 may be connected to the mounting heads 114 by screw. Next, the inner shells 122 may be buckled with the outer shells 121. Finally, the clamping blocks 1222 of the inner shells 122 may be fitted in the clamping ports 1141 of the mounting heads 114, and the electrode assembly 14 may be mounted on the mounting boss 135 of the flexible sleeve 13.

According to the neck massaging device 10 of the present disclosure, by arranging the flexible sleeve 13 to replace the silicon rubber inner shell, which may be directly covered on the carrying body 11 without an additional bonding way, a series of problems brought by glue overflowing mentioned in the background may be avoided. Thus, the assembling efficiency and consumption experience may be greatly improved. Moreover, according to the neck massaging device 10, the outer shell 121 and the inner shell 122 of the handle 12 may be assembled under the assistance of the mounting head 114. During disassembly, the clamping block 1222 of the inner shell 122 may be separated from the mounting head 114 by only outwards pulling apart the handle 12 manually by the slight deformation of the outer shell 121. Therefore, it is very convenient for the neck massaging device 10 to be disassembled.

It may be understood that, in other implementation ways, the carrying body 11 may also be an anti-deformation member, while the pair of handles 12 may be movably hinged to two ends of the carrying body 11. Two torsional springs are arranged between the pair of handles 12 and the carrying body 11 respectively. Therefore, the pair of handles 12 may be clamped on the neck of the wearer by elastic force of the torsional springs.

The above descriptions may be only preferred embodiments of the present disclosure, and thus do not limit the patent claims of the present disclosure. Any equivalent structural transformation made by using the description and drawings of the present disclosure under the concept of the present disclosure, or direct/indirect application in other related technical fields may be included in the patent protection scope of the present disclosure.

## Claims

1. A neck massaging device, comprising: a carrying body, two handles, an electrode assembly and an electric pulse generating device, the two handles connected to each end of the carrying body, the electric pulse generating device electrically connected with the electrode assembly, the electric pulse generating device arranged in one of the handles, **characterized in that**: the neck massaging device further comprises a hollow flexible sleeve which is tensioned over the carrying body, and the electrode assembly is disposed on the flexible sleeve.

2. The neck massaging device according to claim 1, wherein the carrying body is configured into an arc-shaped sheet body having two long sides and two short sides, wherein the carrying body is provided with clamping strips extending along the two long sides of the carrying body, wherein the flexible sleeve is correspondingly provided with clamping grooves in which the clamping strips are embedded.

3. The neck massaging device according to claim 2, wherein there are two clamping strips, the two clamping strips are symmetrically distributed with each being arranged on each long side respectively.

4. The neck massaging device according to claim 2, wherein said two short sides are provided with two mounting heads respectively; wherein the two handles are fixed to the two mounting heads respectively.

5. The neck massaging device according to claim 4, wherein each of the handles comprises an outer shell and an inner shell buckled with the outer shell, the outer shell threadedly connected to the mounting head; and an inner shell further detachably clamped on the mounting head.

6. The neck massaging device according to claim 5, wherein a locking block is protruded on the mounting head, and the flexible sleeve is correspondingly provided with a locking groove in which the locking block is locked respectively.

7. The neck massaging device according to claim 6, wherein the outer shell is provided with a grabbing buckle, and a buckle hole is correspondingly formed in the flexible sleeve, wherein
the grabbing buckle is hooked in the buckle hole.

8. The neck massaging device according to any one of claims 1 to 7, wherein a stepped structure is formed on each end of the flexible sleeve, wherein
the neck massaging device further comprises a decorative ring hooked over the stepped structure and borne against a position between the flexible sleeve and one of the handles.

9. The neck massaging device according to any one of claims 1 to 7, wherein the flexible sleeve is integrally formed with a mounting boss on which the electrode assembly is mounted, and the electrode assembly swings freely by 360-degree in a floating manner via the folding deformation of the mounting boss.

10. The neck massaging device according to any one of claims 1 to 7, wherein the carrying body is an anti-deformation member or an elastic arm.

11. An electrode plate mounting structure, comprising electrode plates, **characterized in that**: the electrode plate mounting structure further comprises a carrying body and a hollow flexible sleeve which tensioned over the carrying body, and the electrode assembly disposed on the flexible sleeve.

12. The electrode plate mounting structure according to claim 11, wherein the flexible sleeve is integrally formed with mounting bosses, on which the electrode plates are mounted respectively; and
the electrode plates swing freely by 360-degree in a floating manner via the folding deformation of the mounting boss.

13. The electrode plate mounting structure according to claim 11, wherein the carrying body is an anti-deformation member or an elastic arm.

14. The electrode plate mounting structure according to claim 11, wherein the carrying body is configured into an arc-shaped sheet body having two long sides and two short sides, wherein
the carrying body is provided with clamping strips extending along the two long sides of the carrying body, and
the flexible sleeve is correspondingly provided with clamping grooves in which the clamping strips are embedded.
